Europäisches Patentamt

(19) European Patent Office

Office européen des brevets

(11) **EP 0 627 908 B1**

(12) **EUROPEAN PATENT SPECIFICATION**

(45) Date of publication and mention
of the grant of the patent:
**11.11.1998 Bulletin 1998/46**

(21) Application number: **93904254.5**

(22) Date of filing: **25.02.1993**

(51) Int. Cl.$^6$: **A61K 7/16**, A61K 7/22

(86) International application number:
**PCT/GB93/00390**

(87) International publication number:
**WO 93/16681 (02.09.1993 Gazette 1993/21)**

(54) **COMPOSITIONS**

ZUSAMMENSETZUNGEN

COMPOSITIONS

(84) Designated Contracting States:
**AT BE CH DE DK ES FR GB GR IE IT LI LU MC NL
PT SE**

(30) Priority: **29.02.1992 GB 9204410**

(43) Date of publication of application:
**14.12.1994 Bulletin 1994/50**

(60) Divisional application:
**98200795.7 / 0 862 909**

(73) Proprietor:
**SMITHKLINE BEECHAM PLC
Brentford, Middlesex TW8 9EP (GB)**

(72) Inventors:
• **BARNETT, Paul,
SmithKline Beecham Consumer Brands
Weybridge, Surrey KT13 0DE (GB)**
• **BURGON-LYON, K.
SmithKline Beecham Consumer Brands
Weybridge, Surrey KT13 0DE DE (GB)**

(74) Representative:
**Connell, Anthony Christopher et al
SmithKline Beecham plc
Corporate Intellectual Property,
Two New Horizons Court
Brentford, Middlesex TW8 9EP (GB)**

(56) References cited:
**DE-A- 3 541 025          FR-A- 1 075 782
FR-A- 2 238 475          GB-A- 1 495 605
US-A- 4 775 525**

Note: Within nine months from the publication of the mention of the grant of the European patent, any person may give
notice to the European Patent Office of opposition to the European patent granted. Notice of opposition shall be filed in
a written reasoned statement. It shall not be deemed to have been filed until the opposition fee has been paid. (Art.
99(1) European Patent Convention).

EP 0 627 908 B1

Printed by Xerox (UK) Business Services
2.16.6/3.4

## Description

The present invention relates to oral hygiene compositions comprising a cationic anti-bacterial agent and an anti-staining agent, for use in treating or preventing dental plaque, caries, or periodontal diseases of the oral cavity in humans or lower animals with reduced staining of teeth.

Cationic antibacterial agents such as chlorhexidine have long been recognised as useful therapeutic agents for treating plaque, caries and peridontal diseases due not only to their intrinsic *in vitro* antibacterial activity against strains implicated in the formation of plaque but also because of their substantivity to oral tissue. They are thus adsorbed onto oral surfaces and gradually released over a period of time, to produce a prolonged antibacterial effect and hence reduce or prevent the formation of plaque over a period of time. It is believed that the deposition of plaque onto the tooth surface is one of the first steps in the development of dental caries and periodontal disease.

It is however also widely recognised that the use of cationic antibacterial agents in oral hygiene, in particular chlorhexidine, is accompanied by the formation of a cosmetically disfiguring brown stain on the tooth surfaces. This is believed to arise from an interaction between the cationic antibacterial agent and certain dietary components, particularly those rich in tannins such as tea, coffee and red wine. Whilst this problem may to some extent be alleviated by brushing of the teeth with a toothpaste prior to use of the product containing the cationic antibacterial agent, in many instances it is found necessary to resort to professionally administered scaling and polishing to remove the stain.

In consequence there have been many suggestions in the patent literature for chemical anti-staining agents which maybe incorporated with the cationic antibacterial agent or which may, in some instances, be used instead separately, after the cationic antibacterial agent. Examples include carboxylic acids (US 4 256 731), aminocarboxylate compounds (US 4 080 441), phosphonoacetic acid (US 4 118 474), peroxydiphosphate salts (US 4 273 759) and, more recently, a polymer having certain pendant polyalkylene oxide chains (GB 2 213 721-A).

We have now surprisingly found that the development of stain may be at least substantially mitigated if not eliminated by the use of another polymeric material *viz* polyvinyl pyrrolidone (PVP). This material is already used in some toothpastes at comparatively low levels (typically about 0.1%) as an auxiliary thickening agent and foam enhancer. In addition, it has been previously reported that PVP may be used in oral hygiene compositions, for the prevention of stains associated with the use of chlorophyll in such compositions (GB 739 936, Colgate-Palmolive Co) and also in the removal of stains, in particular tobacco tar stains, from tooth surfaces (GB 741 315, Colgate-Palmolive-Peet Co).

Accordingly, the present invention provides a mouthwash composition comprising a bacteriostatic effective amount of chlorhexidine or an orally acceptable acid addition salt thereof an anti-stain effective amount of polyvinyl pyrrolidone, a non-ionic, cationic or amphoteric surfactant and an orally acceptable carrier or excipient.

Polyvinyl pyrrolidone for use in the present invention suitably has an average molecular weight in the range 5,000 to 100,000, preferably in the range 5,000 to 50,000. Polyvinyl pyrrolidones which have average molecular weights of 10,000, 30,000 and 40,000 are available from Sigma Chemical Co., GAF Corporation and Sigma Chemical Co. respectively. Polyvinyl pyrrolidone is suitably present in at least 1%, preferably between 2 and 30%, more preferably between 5 and 25%, and advantageously between 10 and 25% by weight of the composition.

Suitably, the chlorhexidine or salt thereof is present in from 0.005 to 10% prefeably 0.01 to 5%, more preferably 0.01 to 2%, by weight of the mouthwash composition.

Suitable such salts of chlorhexidine include those which have a solubility at 20°C of at least 0.005% w/v and include the digluconate, diformate, diacetate, dipropionate, dihydroiodide, dihydrochloride, dilactate, dinitrate, sulphate, and tartrate. Of these, the digluconate, diacetate and dihydrochloride are favoured.

Mouthwash compositions of the present invention may also usefully contain an ionic fluorine-containing compound. Suitable ionic fluorine-containing compounds include, for instance, fluoride salts such as amine fluorides and alkali metal fluoride salts, for example sodium fluoride, and monofluorophosphate salts such as alkali metal monofluorophosphate salts, for example sodium monofluorophosphate. Suitably the ionic fluorine-containing compound is incorporated into the composition to provide between 100 and 3000ppm, preferably between 500 and 2000ppm of fluoride ions.

It will be appreciated by those skilled in the art that in order to ensure that the antibacterial efficacy of chlorhexidine is not substantially diminished, compatible components will be selected for inclusion in the orally acceptable carrier or excipient. Accordingly, the skilled man will readily appreciate that where necessary anionic species should be preferably avoided as such species may cause inactivation of the cationic antibacterial agent by the formation of an insoluble precipitate therewith. Thus, anionic surfactants and anionic thickening agents should preferably be rejected in favour of nonanionic counterparts such as nonionic, cationic or amphoteric surfactants and nonionic thickening agents.

Suitable compositions comprising chlorhexidine are described in EP 0 364 245-A2, EP 0 368 130-A2 and EP 0 422 803-A2.

Suitable nonionic surfactants include, for example, polyethoxylated sorbitol esters, in particular polyethoxylated sorbitol monoesters, for instance, PEG(40) sorbitan di-isostearate, and the products marketed under the trade name 'Tween' by ICI; polycondensates of ethylene oxide and propylene oxide (poloxamers), for instance the products marketed under the trade name 'Pluronic' by BASF-Wyandotte; condensates of propylene glycol; polyethoxylated hydrogenated castor oil, for instance, cremophors; and sorbitan fatty esters.

Suitable amphoteric surfactants include, for example, long chain imidazoline derivatives such as the product marketed under the trade name 'Miranol C2M' by Miranol; long chain alkyl betaines, such as the product marketed under the tradename 'Empigen BB' by Albright + Wilson, and long chain alkyl amidoalkyl betaines, such as cocamidopropylbetaine, and mixtures thereof.

Suitable cationic surfactants include the D,L-2-pyrrolidone-5-carboxylic acid salt of ethyl-N-cocoyl-L-arginate, marketed under the trade name CAE by Ajinomoto Co. Inc.

Advantageously, the surfactant is present in the range 0.005 to 20%, preferably 0.1 to 10%, more preferably 0.1 to 5% by weight of the dentifrice.

Suitable nonionic thickening agents include, for example, $(C_{1-6})$-alkylcellulose ethers, for instance methylcellulose, hydroxy$(C_{1-6})$-alkylcellulose ethers, for instance hydroxypropylcellulose, $(C_{2-6})$-alkylene oxide modified $(C_{1-6})$-alkylcellulose ethers, for instance hydroxypropyl methylcellulose, and mixtures thereof. Advantageously the non-ionic thickening agent is present in the range 0.01 to 30%, preferably 0.1 to 15%, more preferbly 1 to 5%, by weight of the composition.

Mouthwashes according to the present invention will preferably comprise as components of the carrier a surfactant and a humectant in an aqeueous/ethanol solution.

Suitable humectants for use in compositions of the invention include for instance glycerine, sorbitol, propylene glycol or polyethylene glycol, or mixtures thereof. The humectant may be present in the range from 5 to 70%, preferably 5 to 30%, more preferably 10 to 30% by weight of the dentifrice.

Other materials may be added to the compositions if required, for instance sweetening agents, flavouring agents, colouring and whitening agents, preservatives and emulsifiers.

The pH of a composition according to the invention will be orally acceptable and typically in the range pH 5 to 9.

Mouthwash compositions according to the present invention may be prepared by mixing the ingredients thereof in the required proportions and in any order that is convenient and thereafter and if necessary adjusting the pH to the required value.

Mouthwash compositions according to the present invention are of use in reducing or eliminating the stain normally asociated with the use of a chlorhexidine. Accordingly, in a further aspect, the present invention provides for a mouthwash composition as hereinbefore defined for use in therapy, in particular anti-plaque, anti-caries, anti-calculus and/or periodontal (including anti-gingivitis) therapy. The present invention also provides for the use of polyvinyl pyrrolidone and a chlorhexidine agent in the manufacture of a mouthwash composition for use in oral hygiene.

In mouthwash compositions according to the present invention, chlorhexidine and the polyvinyl pyrrolidone anti-stain agent will normally be incorporated together in a single composition. Chlorhexidine and the polyvinylpyrrolidone anti-stain agent may however be also provided in separate mouthwash compositions which may be used separately, simultaneously or sequentially. Accordingly, in a further aspect, the present invention provides a mouthwash kit comprising as a first item a mouthwash composition comprising an anti-stain effective amount of polyvinyl pyrrolidone and an orally acceptable excipient or carrier and, as a separate second item, a mouthwash composition comprising a bacteriostatic effective amount of chlorhexidine or an orally acceptable acid addition salt thereof and an orally acceptable excipient or carrier wherein said compositions further comprise a non-ionic, cationic or amphoteric surfactant.

The invention will now be illustrated by reference to the following examples.

**Example 1 - Efficacy of PVP in a stain model -** The efficacy of polyvinylpyrrolidone (PVP) in preventing the formation of stain associated with the use of chlorhexidine was evaluated *in vitro* in a model system which consisted of exposing a bovine incisor to a tea solution and then treating this with chlorhexidine in the absence or presence of polyvinylpyrrolidone.

A bovine incisor was mounted in an acrylic block and cleaned on the exposed tooth surface. The colour of this incisor was measured using a chromameter (Minolta CR200) to establish a baseline figure. The incisor was then rinsed with distilled water for 1 min and incubated in pooled filtered human saliva for 2 hour at 37°C, to induce pellicle formation. After further rinsing for 1 min with distilled water, the incisor was immersed for 2 min in a solution containing chlorhexidine digluconate (1% in 0.1M sodium acetate, pH 5.6), after which the incisor was rinsed with distilled water (1 minute) and then immersed in cold tea for 3 hr. The incisor was then rinsed with distilled water for 1 min, immersed in pooled filtered human saliva for 1 hr at 37°C, rinsed with distilled water for 1 min, subjected to a 2 min application of chlorhexidine (1%), rinsed with distilled water for 1 min and then immersed in cold tea overnight. The procedure was repeated daily for 7 days. At the end of the 7 days, the colour of the tooth was determined using the chromameter. The degree of colour change was calculated using the formula:

$$E^* ab = \sqrt{[\Delta L^2 + \Delta a^2 + \Delta b^2]}$$

in which    $E^* ab$ is colour difference
$L$ is degree of black/white colour
$a$ is degree of green/red colour
$b$ is degree of blue/yellow colour.

In each treatment group, 5 incisors were used.

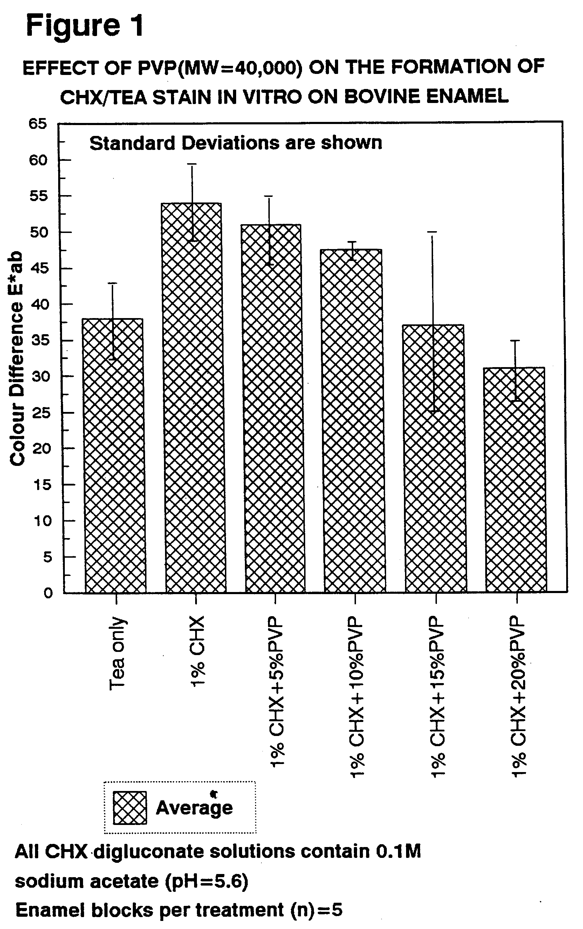

The procedure was then repeated, but using a solution containing chlorhexidine (1%) and PVP (av. mol. wt. 40,000, at 5, 10, 15 and 20%) instead of chlorhexidine alone. In addition, a control was run in which the molar was treated with neither chlorhexidine or PVP. The results are presented graphically as Figure 1. The solutions comprising 15 and 20% concentrations of PVP were found to be totally effective in reducing the formation of the CHX/tea stain. There was also a trend towards efectiveness at lower levels of PVP.

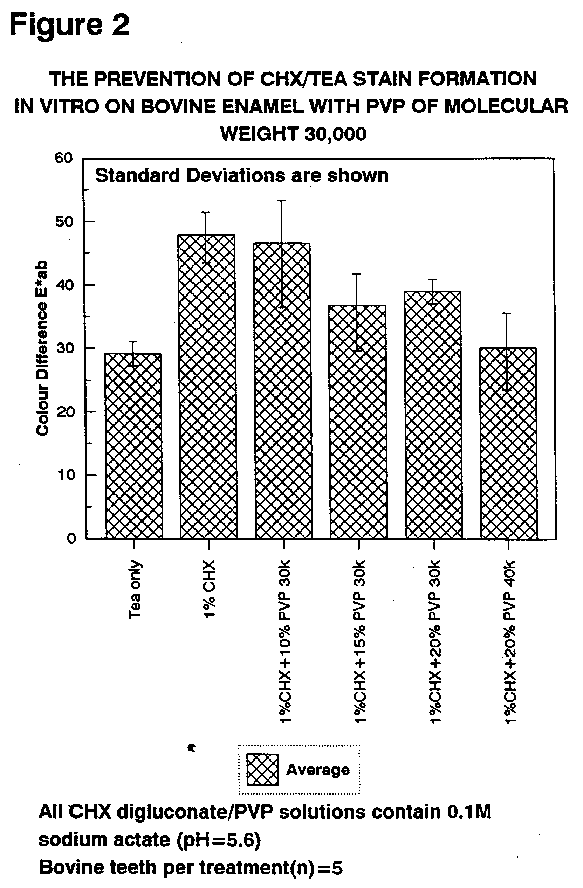

In a second set of experiments, solutions containing chlorhexidine digluconate (1%) and various amounts of PVP (M W 30000 - 10, 15 and 20%;and M W 40000 - 20%) in sodium acetate (0.1M) at pH 5.6 were tested as above. The results are presented graphically as Figure 2. The treatment of bovine enamel with PVP (20%, MW = 40,000; 15%, MW = 30,000; 20%, MW = 30,000) was found to result in significantly less stain formation than treatment with chlorhexidine alone. The reduction in stain using PVP (10%, MW = 30,000) was not however significantly significant.

**Example 2** - The impact of polyvinylpyrrolidone on the antibacterial activity of chlorhexidine was evaluated as follows:

Solutions of chlorhexidine (1%) were tested in the presence and absence of PVP (10%, molecular weights of 10,000, 30,000 and 40,000) for microbiological activity using *S. sanguis* as the test organism. Polyvinyl pyrrolidone alone was found to have no antibacterial activity. PVP (10% solution, Mol wt. 30,000 and 40,000) had no significant effect on the activity of the chlorhexidine whilst PVP(10% solution, mol wt 10,000) reduced activity by 12% (significance p=0.019).

When the assessment was extended to higher concentrations of PVP, PVP (15 and 20%, mol wt 30,000 and 40,000) was found to have a significant effect on the activity of chlorhexidine against the test organism *S.sanguis*. PVP (mol wt 30,000) (15% and 20%) reduced activity by 20% and 28% respectively. PVP (mol wt 40,000) (15 and 20%) reduced activity by 38% and 32% respectively. Against the test organism *B. ureolyticus,* PVP (15 and 20%)(mol wt 30,000) significantly reduced activity by 26 and 20% respectively. PVP (mol wt 40000) significantly reduced activity by 14% when tested at 20% but not at 15%.

**Claims**

1. A mouthwash composition comprising a bacteriostatic effective amount of chlorhexidine or an orally acceptable acid addition salt thereof, an anti-stain effective amount of polyvinyl pyrrolidone, a non-ionic, cationic or amphoteric surfactant and an orally acceptable carrier or excipient.

2. A composition as claimed in claim 1 in which polyvinyl pyrrolidone has an average molecular weight in the range 5,000 to 100,000.

3. A composition as claimed in claim 1 or 2 in which polyvinyl pyrrolidone is present in from at least 1% by weight of the composition.

4. A composition as claimed in any one of claims 1 to 3 in which polyvinyl pyrrolidone is present in from 2 to 30% by weight of the composition.

5. A composition as claimed in any one of claims 1 to 4 further comprising an ionic fluorine-containing compound.

6. A composition as defined in any one of claims 1 to 5 in which the surfactant comprises a non-ionic surfactant.

7. A composition as defined in any one of claims 1 to 6 for use in therapy.

8. The use of polyvinyl pyrrolidone and chlorhexidine or an orally acceptable acid addition salt thereof in the manufacture of a mouthwash composition with reduced staining.

9. A mouthwash kit comprising as a first item a mouthwash composition comprising an anti-stain effective amount of polyvinyl pyrrolidone and an orally acceptable carrier or excipient and, as a separate second item, a mouthwash composition comprising a bacteriostatic effective amount of chlorhexidine or an orally acceptable acid addition salt thereof and an orally acceptable carrier or excipient, wherein said compositions further comprise a non-ionic, cationic or amphoteric surfactant.

10. The use of polyvinyl pyrrolidone in the manufacture of a mouthwash composition for use, separately, simultaneously or sequentially, with a mouthwash composition comprising a bacteriostatic effective amount of chlorhexidine or an orally acceptable acid addition salt thereof, to reduce or eliminate the stain associated with the use of a mouthwash composition comprising chlorhexidine.

11. A process for preparing a mouthwash composition as defined in any one of claims 1 to 6 which process comprises admixing the ingredients in the appropriate amounts in any order that is convenient and, if necessary, adjusting the pH to the final required value.

## Patentansprüche

1. Mundwasserzusammensetzung, umfassend eine bakteriostatisch wirksame Menge an Chlorhexidin oder einem oral verträglichen Säureadditionssalz davon, eine gegen Verfärbung wirksame Menge an Polyvinylpyrrolidon, ein nicht-ionisches, kationisches oder amphoteres grenzflächenaktives Mittel sowie einen oral verträglichen Träger oder Excipienten.

2. Zusammensetzung nach Anspruch 1, in der Polyvinylpyrrolidon ein mittleres Molekulargewicht im Bereich von 5000 bis 100000 aufweist.

3. Zusammensetzung nach Anspruch 1 oder 2, in der Polyvinylpyrrolidon mit wenigstens 1 Gew.% der Zusammensetzung vorhanden ist.

4. Zusammensetzung nach einem der Ansprüche 1 bis 3, in der Polyvinylpyrrolidon mit 2 bis 30 Gew.% der Zusammensetzung vorhanden ist.

5. Zusammensetzung nach einem der Ansprüche 1 bis 4, ferner umfassend eine ionische fluorhaltige Verbindung.

6. Zusammensetzung nach einem der Ansprüche 1 bis 5, in der das grenzflächenaktive Mittel ein nicht-ionisches grenzflächenaktives Mittel umfaßt.

7. Zusammensetzung nach einem der Ansprüche 1 bis 6 zur Verwendung in der Therapie.

8. Verwendung von Polyvinylpyrrolidon und Chlorhexidin oder einem oral verträglichen Säureadditionssalz davon bei der Herstellung einer Mundwasserzusammensetzung mit verminderter Verfärbung.

9. Mundwasserset, umfassend als ersten Gegenstand eine Mundwasserzusammensetzung, umfassend eine gegen Verfärbung wirksame Menge an Polyvinylpyrrolidon und einen oral verträglichen Träger oder Excipienten, sowie als zweiten separaten Gegenstand eine Mundwasserzusammensetzung, umfassend eine bakteriostatisch wirksame Menge an Chlorhexidin oder einem oral verträglichen Säureadditionssalz davon und einen oral verträglichen Träger oder Excipienten, wobei die Zusammensetzungen ferner ein nicht-ionisches, kationisches oder amphoteres grenzflächenaktives Mittel umfassen.

10. Verwendung von Polyvinylpyrrolidon bei der Herstellung einer Mundwasserzusammensetzung zur getrennten, gleichzeitigen oder sequentiellen Verwendung mit einer Mundwasserzusammensetzung, umfassend eine bakteriostatisch wirksame Menge an Chlorhexidin oder einem oral verträglichen Säureadditionssalz davon, um die Verfarbung, die mit der Verwendung einer Mundwasserzusammensetzung, umfassend Chlorhexidin, verbunden ist, zu vermindern oder zu beseitigen.

11. Verfahren zur Herstellung einer Mundwasserzusammensetzung nach einem der Ansprüche 1 bis 6, wobei das Verfahren umfaßt: Mischen der Bestandteile in den passenden Mengen in jeder beliebigen Reihenfolge, die günstig ist, und gegebenenfalls Einstellen des pH-Werts auf den erforderlichen Endwert.

## Revendications

1. Composition de bain de bouche comprenant une quantité efficace du point de vue bactériostatique, de chlorhexidine ou d'un sel d'addition d'acide de celle-ci oralement acceptable, une quantité efficace du point de vue anticoloration de polyvinylpyrrolidone, d'un surfactant non ionique, cationique ou amphotère et un véhicule ou excipient oralement acceptable.

2. Composition selon la revendication 1, dans laquelle la polyvinylpyrrolidone a un poids moléculaire moyen compris entre 5.000 et 100.000.

3. Composition selon la revendication 1 ou la revendication 2, dans laquelle la polyvinylpyrrolidone est présente à raison d'au moins 1% en poids par rapport à la composition.

4. Composition selon l'une quelconque des revendications 1 à 3, dans laquelle la polyvinylpyrrolidone est présente à raison de 2 à 30% en poids par rapport à la composition.

5. Composition selon l'une quelconque des revendications 1 à 4, comprenant un composé ionique contenant du fluor.

6. Composition selon l'une quelconque des revendications 1 à 5, dans laquelle le surfactant comprend un surfactant non ionique.

7. Composition selon l'une quelconque des revendications 1 à 6, en vue de l'utilisation en thérapie.

8. Utilisation de polyvinylpyrrolidone ou de chlorhexidine ou d'un sel d'addition d'acide oralement acceptable de celle-ci, dans la fabrication d'une composition de bain de bouche de coloration réduite.

9. Kit de bain de bouche comprenant en tant que premier élément, une composition de bain de bouche contenant une quantité de polyvinylpyrrolidone efficace du point de vue anticoloration et un véhicule ou excipient oralement acceptable, et en tant que second élément, une composition de bain de bouche contenant une quantité efficace du point de vue bactériostatique, de chlorhexidine ou d'un sel d'addition d'acide de celle-ci oralement acceptable, et un véhicule ou excipient oralement acceptable, dans lequel lesdites compositions comprennent un surfactant non ionique, cationique ou amphotère.

10. Utilisation de polyvinylpyrrolidone dans la fabrication d'une composition de bain de bouche pour l'emploi en mode séparé, simultané ou séquentiel avec une composition de bain de bouche contenant une quantité efficace du point de vue bactériostatique, de chlorhexidine ou d'un sel d'addition d'acide oralement acceptable de celle-ci, pour réduire ou éliminer la coloration associée à l'emploi d'une composition de bain de bouche comprenant de la chlorhexidine.

11. Procédé de préparation d'une composition de bain de bouche selon l'une quelconque des revendications 1 à 6, lequel procédé comprend l'opération de mélange des ingrédients en quantités appropriées, pris dans un ordre quelconque jugé commode et si nécessaire, l'ajustage du pH à la valeur finale requise.

# Figure 1

## EFFECT OF PVP(MW=40,000) ON THE FORMATION OF CHX/TEA STAIN IN VITRO ON BOVINE ENAMEL

All CHX digluconate solutions contain 0.1M
sodium acetate (pH=5.6)
Enamel blocks per treatment (n)=5

7

# Figure 2

## THE PREVENTION OF CHX/TEA STAIN FORMATION IN VITRO ON BOVINE ENAMEL WITH PVP OF MOLECULAR WEIGHT 30,000

**All CHX digluconate/PVP solutions contain 0.1M sodium actate (pH=5.6)**
**Bovine teeth per treatment(n)=5**